Europäisches Patentamt

(19) European Patent Office

Office européen des brevets

(11) Veröffentlichungsnummer: **0 046 167 B1**

(12) **EUROPÄISCHE PATENTSCHRIFT**

(45) Veröffentlichungstag der Patentschrift:
12.12.84

(51) Int. Cl.³: **A 61 K 37/18, A 61 K 31/205**

(21) Anmeldenummer: 81103647.4

(22) Anmeldetag: 12.05.81

(54) **Nährlösung für die vollständige parenterale Ernährung und für gesteigerte Energieproduktion und Verfahren zu deren Herstellung.**

(30) Priorität: 11.07.80 DE 3026368

(43) Veröffentlichungstag der Anmeldung:
24.02.82 Patentblatt 82/8 .

(45) Bekanntmachung des Hinweises auf die Patenterteilung:
12.12.84 Patentblatt 84/50

(84) Benannte Vertragsstaaten:
AT BE CH DE FR GB IT LI LU NL SE

(56) Entgegenhaltungen:
DE - A - 2 921 312
GB - A - 2 059 262
US - A - 3 810 994

UNLISTED DRUGS, Juni 1978, CHATHAM, N.J. (US)
Hager Handbuch der Pharmazeutischen Praxis B VII Teil
A 1971 Seite 401-405, 424-426

Die Akte enthält technische Angaben, die nach dem
Eingang der Anmeldung eingereicht wurden und die
nicht in dieser Patentschrift enthalten sind.

(73) Patentinhaber: Leopold & Co. Chem. pharm. Fabrik
Gesellschaft m.b.H., Hafnerstrasse 36, A-8055 Graz (AT)

(84) Benannte Vertragsstaaten: BE CH FR GB IT LI LU NL SE
AT

(73) Patentinhaber: Lentia Gesellschaft mit beschränkter
Haftung, Arabellastrasse 4 Postfach 81 05 08,
D-8000 München 81 (DE)

(84) Benannte Vertragsstaaten: DE

(72) Erfinder: Jeretin, Stojan, Dr., Ferberjeva 37,
YU-6100 Ljubljana (YU)
Erfinder: Groke, Karl, Dr., A-8063 Eggersdorf 86 (AT)
Erfinder: Musil, Horst-Eberhard, Dr., Hans Riehlgasse 8,
A-8043 Graz (AT)

(74) Vertreter: Allgeuer, Dorothea, Chemie Linz AG
Patentabteilung St. Peter-Strasse 25, A-4020 Linz (AT)

**Beschreibung**

Die vorliegende Erfindung betrifft eine aminosäurehaltige Nährlösung für die vollständige parenterale Ernährung und Behandlung von Patienten in der Postaggressionsphase, die eine ausreichende Aminosäureversorgung des Körpers und eine gesteigerte Energieproduktion gewährleistet.

Die parenterale Ernährung von Patienten, die aus irgendwelchen Gründen keine Nahrung zu sich nehmen können, gewinnt immer mehr an Bedeutung. Dabei ist man bestrebt, nicht nur den Kohlehydratbedarf des Körpers zu decken, sondern auch durch Zufuhr von Aminosäuren die Funktions- und Strukturproteine zu erhalten. In Hagers Handbuch der Pharmazeutischen Praxis B VII, Arzneiformen und Hilfsstoffe, Teil A, Springer Verlag 1971, Seite 401 bis 405 und 424 bis 426, sind die Grundzüge der parenteralen Ernährung zusammengefaßt, wobei, basierend auf Untersuchungen am Gesunden, der minimale Tagesbedarf an den essentiellen Aminosäuren sowie die empfehlenswerte Menge in g, die verabreicht werden soll, angegeben wird. Für die nichtessentiellen Aminosäuren, denen vor allem die Rolle einer unspezifischen Stickstoffquelle zugeschrieben wird, sind hingegen nur qualitative Hinweise gegeben. Auf Seite 426 wird der Mindestbedarf an Aminosäuren pro Tag und kg Körpergewicht mit 0,8 bis 1 g angegeben.

Bei Patienten in der Postaggressionsphase, d. h. also nach Unfällen, oder im postoperativen Zustand, ist zur Kompensation der gesteigerten Proteolyse ein höherer Bedarf an Aminosäuren, z. B. von 1,5—2 g Aminosäuren pro kg Körpergewicht und Tag gegeben. Verabreicht man aber einfach Aminosäuren in dieser Menge, ist deren Einbau in das körpereigene Protein nicht gewährleistet, da dazu Energie, nämlich für den Einbau von 1 g Stickstoff 120—200 kcal, erforderlich ist, die gerade bei schwerkatabolen Patienten nicht zur Verfügung steht.

Dieser Situation Rechnung tragend, ist es daher schon bekannt, daß zusätzlich zu den Aminosäuren Energieträger verabreicht werden müssen, siehe z. B. H. Förster, Internist 19, 2—19 (1978). Als solche Energieträger dienen Kohlehydrate, Fette, Alkohol, wobei meist Kombinationen eingesetzt werden, da jeder der drei Energieträger, vor allem in zu hoher Dosierung nachteilig wirkt.

So ist z. B. Glucose als allgemeiner Energieträger nur dann wirksam, wenn mehr verabreicht wird als der Glucosebedarf der ausschließlich glucoseabhängigen Körpergewebe ausmacht. Dieser beträgt üblicherweise 120—150 g, maximal 200 g/Tag. Als Energielieferant müssen also mehr als 200 g Glucose/Tag gegeben werden. Derart hohe Dosen können aber, vor allem bei Patienten in der Postaggressionsphase, zu einer Hyperglykämie und zur Ausscheidung von Glucose über den Harn bzw. auch zur Erhöhung der Laktatkonzentration im Blut führen. Besser verträglich sind höhere Mengen an Fructose, doch kann auch deren Verabreichung die Erhöhung der Laktatkonzentration zur Folge haben. Sorbit verhält sich ähnlich wie Fructose und ist zudem in hoher Konzentration osmotisch wirksam. Allen Ersatzzuckern (Fructose, Sorbit, Xylit) ist die erhöhte Phosphorylierungsrate mit Abfall der Serumphosphatkonzentration sowie ein möglicher Anstieg der Serum-Harnsäurekonzentration gemeinsam.

Der Hauptenergieträger ist daher in den Fetten zu suchen, deren Verabreichung aber auch nicht komplikationslos möglich ist. Sie führt z. B. zu Störungen in der Fettverwertung, die wiederum die Ursache für einen starken Anstieg der Fettkonzentration im Serum ist, die eine Leberschädigung und Blockade des Retikuloendothelialen Systems nach sich ziehen kann.

Äthylalkohol als Energiequelle einzusetzen, ist überhaupt problematisch und bei Leberschäden kontraindiziert, obwohl er ohne Zweifel einen hohen Energiegehalt besitzt. Der Hauptnachteil besteht im hohen osmotischen Druck, der wiederum zu Thrombophlebitiden führen kann. Es gab also für die Energieversorgung bei der parenteralen Ernährung noch keine befriedigende Lösung, sondern es mußten oft gravierende Nachteile in Kauf genommen werden.

Es ist andererseits bekannt, daß der Naturstoff, Carnitin, nämlich β-Hydroxy-trimethylammoniumbutyrobetain neben anderen pharmakologisch auswertbaren Wirkungen auch eine Wirkung auf den Feststoffwechsel in der Weise besitzt, daß damit die Lipidkonzentration in Blut gesenkt wird. Diese Wirkung beruht darauf, daß Carnitin den Transport von Fettsäuren in die Mitochondrien begünstigt. Carnitin wurde daher schon zur Bekämpfung von Fettleibigkeit eingesetzt, wobei eine Gewichtsreduktion vor allem dann erzielbar ist, wenn gleichzeitig diätetische Maßnahmen ergriffen werden (US-A 3 810 994). Gemäß DE-A 2 323 906 ist die Wirkung von Carnitin allein zum Abbau von Fettdepots bzw. zur Senkung des Lipidspiegels und zur Verhütung von Degenerationserscheinungen, wie Arteriosklerose, zu gering, so daß die gleichzeitige Gabe von sog. »fettspaltenden Agenten«, empfohlen wird. Andererseits diente es auch schon zur Verbesserung der Verwertung von parenteral verabreichten Fettemulsionen, siehe BE-PS 639 532 und DE-OS 2 921 312.

Es konnte nun überraschenderweise gefunden werden, daß es möglich ist, für die vollständige parenterale Ernährung, insbesondere in der Postaggressionsphase, Gesamtnährlösungen zu schaffen, die eine optimale Ausnützung der verabreichten Aminosäuren ermöglichen, weil gleichzeitig die nötige Energiemenge vorhanden ist, ohne daß die oben geschilderten Nachteile in Kauf genommen werden müssen. Diese Lösungen enthalten also keine Fettemulsionen und Glucose oder Glucosersatzstoffe nur in jenem Ausmaß, als sie zur Kohlenhydratversorgung des Körpers notwendig sind. Daß trotzdem durch die erfindungsgemäßen Lösungen die für den Einbau der Aminosäuren nötige Energie bereitgestellt wird, beruht darauf, daß pro Gewichtsteil Aminosäuren eine gewisse Menge an L-Carnitin zugegen ist, das vor allem in der Postaggressionsphase in der Lage ist, durch den Abbau der aus

2

körpereigenen Depots in die Blutbahn im Übermaß eingeschwemmten Fettsäuren (katecholamininduzierte Lipolyse) die nötige Energie bereit zu stellen.

Gegenstand der vorliegenden Erfindung ist demnach eine Nährlösung für die vollständige parenterale Ernährung und für gesteigerte Energieproduktion, insbesondere zur ausreichenden Aminosäureversorgung in der Postaggressionsphase mit einem Gehalt an Zucker, Aminosäuren und L-Carnitin, die dadurch gekennzeichnet ist, daß sie in wäßriger Lösung auf 25 Gewichtsteile eines Aminosäuregemisches, bestehend aus

| | |
|---|---|
| 0,8—1,05 Gewichtsteilen | L-Isoleucin |
| 1,4—1,5 Gewichtsteilen | L-Leucin |
| 1,4—1,65 Gewichtsteilen | L-Lysin |
| 0,5—1,2 Gewichtsteilen | L-Methionin |
| 1,25—1,4 Gewichtsteilen | L-Phenylalanin |
| 0,7—1,05 Gewichtsteilen | L-Threonin |
| 0,35—0,375 Gewichtsteilen | L-Tryptophan |
| 1,0—1,5 Gewichtsteilen | L-Valin |
| 2,0—2,8 Gewichtsteilen | L-Arginin |
| 1,0—1,5 Gewichtsteilen | L-Histidin |
| 3,5—4,0 Gewichtsteilen | L-Alanin |
| 0,35—0,55 Gewichtsteilen | L-Asparaginsäure |
| 0,1 Gewichtsteilen | L-Cystein |
| 2,0—2,25 Gewichtsteilen | L-Glutaminsäure |
| 1,5—2,3 Gewichtsteilen | Glycin |
| 0—0,625 Gewichtsteilen | L-Ornithin |
| 2,2—2,8 Gewichtsteilen | L-Prolin |
| 1,1—1,4 Gewichtsteilen | L-Serin |
| 0—1,5 Gewichtsteilen | L-Tyrosin, |

wobei die Aminosäuren L-Tyrosin und L-Cystein in der Lösung in Form von Acylderivaten vorliegen, maximal 50 Gewichtsteile Glucose oder Glucoseersatzstoffe, wie Fructose und Zuckeralkohole oder eines Gemisches von Glucose und Glucoseersatzstoffen und 0,8—1,2 Gewichtsteile L-Carnitin in Form des inneren Salzes oder eines pharmazeutisch verträglichen Säureadditionssalzes enthält.

Die Angabe der Bereiche ist so zu verstehen, daß die Mengen der einzelnen Aminosäuren innerhalb der angegebenen Grenzen gewählt werden, wobei aber darauf Bedacht zu nehmen ist, daß insgesamt 25 g an Aminosäuregemisch resultieren müssen.

Die erfindungsgemäße Nährlösung kann in jeder, aufgrund der Löslichkeitsverhältnisse möglichen Konzentration formuliert sein, d. h. sie kann als 3%ige, 4%ige, ebensogut aber auch als 10%ige Lösung, berechnet in bezug auf den Aminosäuregehalt, vorliegen. Die 10%ige Lösung enthält also dann pro Liter 100 g Aminosäuren sowie 3,2—4,8 g Carnitin. Wesentlich ist bei allen Konzentrationsstufen nur, daß die erfindungsgemäße Relation von 25 Gew.-Teilen Aminosäuregemisch auf 0,8—1,2 Gew.-Teile Carnitin erfüllt ist. Die schwerlöslichen Aminosäuren Tyrosin und Cystein sind in Form von Acylderivaten und insbesondere als Acetylderivate in der erfindungsgemäßen Lösung enthalten.

Als pharmazeutisch verträgliches Salz des Carnitins, das verabreicht werden kann, sind z. B. das Hydrochorid, das Acetat und das Malat zu nennen.

Die erfindungsgemäße Grenze an Glucose und/oder Glucoseersatzstoffen mit maximal 50 Gewichtsteilen pro 25 Gewichtsteilen Aminosäuregemisch ist so berechnet, daß die Menge in 1 l einer 10%igen Lösung, bezogen auf Aminosäuregehalt, gerade jenen Mengen entspricht, die als Maximalwert für den körpereigenen Bedarf pro Tag der nur mehr unter Ausnahmebedingungen zutrifft, entspricht, bzw. daß sie den normalen körpereigenen Bedarf sicher überschreitet. Dies wurde deshalb gemacht, da es gerade in der Postaggressionsphase wesentlich ist, daß die glucoseabhängigen Organe ausreichend mit diesem Stoff versorgt werden. Natürlich kann die Glucosemenge bzw. die Menge an Glucoseersatzstoffen auch niedriger gehalten werden, wobei die Menge zweckmäßig den verschiedenen Indikationen angepaßt werden kann. Ebenso ist jede beliebige Variation zwischen der Wahl von Glucose, Fructose oder Zuckeralkoholen, wie Sorbit oder Xylit, oder aber von Gemischen dieser Substanzen möglich.

Es kann gerade in der Postaggressionsphase, vor allem zur Aufrechterhaltung der Homöostase, günstig sein, gleichzeitig mit der Nährlösung auch Elektrolyte zuzuführen. Sie dienen einerseits z. B. der Aufrechterhaltung des Säure-Basengleichgewichtes, andererseits ist Kalium für den Proteinaufbau unbedingt erforderlich. Daneben bestehen natürlich auch noch eine Reihe anderer bekannter Vorteile.

Zweckmäßig enthält die erfindungsgemäße Lösung pro 25 Gew.-Teile Aminosäuren 2,5 Gew.-Teile an Elektrolyten. Bevorzugt sind Nà+, Ca++, Mg++, Zn++ und K+ sowie Chlorion und Acetation enthalten.

Die erfindungsgemäße Nährlösung ist nicht nur als Nährlösung gedacht, sondern ist auch in einer Weise zusammengesetzt, die eine Besserung des Allgemeinzustandes des Patienten, vor allem in der Postaggressionsphase, also nach Unfällen, Verbrennungen, Operationen usw. ermöglicht, so daß damit auch eine Behandlung dieses Zustandes gegeben ist. Diese Besserung wird dadurch erzielt, daß durch den erfindungsgemäßen Carnitingehalt freie Fettsäuren in die Mitochondrien eingeschleust werden und damit der Umsatz an Triglyceriden erhöht wird. Dadurch wird ein erhöhter Energieanfall erzielt und außerdem werden durch die Zufuhr von Aminosäuren, die wegen des gleichzeitigen, ausreichenden Energieanfalles vom Körper auch sinnrichtig verwertet werden können, ausreichend Substrate für den energieliefernden Citratcyclus bereitgestellt. Da die allgemeine Energieversorgung durch die Mobilisierung der Fettdepots erfolgt, steht die zugeführte Glucose vollständig dem Bedarf der glucoseabhängigen Gewebe zur Verfügung. Alles dies trägt zur rascheren Wiederherstellung des Patienten bei.

Die erfindungsgemäße Lösung ist daher als Infusionslösung zur parenteralen Ernährung und Behandlung von Patienten in der Postaggressionsphase überall dort angezeigt, wo der Patient noch über körpereigene Fettdepots verfügt, die mobilisiert werden können. Fehlen diese Fettdepots, so kann die erfindungsgemäße Lösung ihre Wirkung natürlich nicht entfalten. Gerade in der Behandlung nach Unfällen, Verbrennungen und anderen traumatischen Zuständen kann aber mit dem Vorhandensein solcher Fettdepots gerechnet werden, so daß gerade in solchen Fällen, die für die bisher übliche parenterale Ernährung wegen der Stoffwechselentgleisung (Lipolyse, Glucoseverwertungsstörung usw.) besonders kritisch sind, die Behandlung mit der erfindungsgemäßen Lösung besonders angezeigt ist.

Die nachfolgenden Beispiele dienen der näheren Erläuterung der vorliegenden Erfindung.

Beispiel 1

Es wird eine Nährlösung mit einer Aminosäurekonzentration von 10% aus folgenden Aminosäuren bereitet:

| L-Isoleucin | 4,200 g |
|---|---|
| L-Leucin | 5,600 g |
| L-Lysin | 5,600 g |
| L-Methionin | 4,800 g |
| L-Phenylalanin | 5,600 g |
| L-Threonin | 2,800 g |
| L-Tryptophan | 1,400 g |
| L-Valin | 4,200 g |
| L-Arginin | 11,200 g |
| L-Histidin | 4,200 g |
| L-Alanin | 14,000 g |
| L-Asparaginsäure | 1,400 g |
| L-Cystein | 0,390 g |
| L-Glutaminsäure | 8,000 g |
| Glycin | 9,200 g |
| L-Prolin | 11,200 g |
| L-Serin | 5,600 g |

Diese Aminosäuren werden zusammen mit 4,000 g L-Carnitin unter Rühren in 750 ml warmem aqua ad infundi gelöst, wobei das L-Cystein als N-Acetyl-L-cystein entsprechend der oben angegebenen Cysteinmenge und L-Lysin in der entsprechenden Menge als L-Lysin-L-glutamat-$H_2O$ eingesetzt wurde. Dieser Lösung wurden 75,000 g Glucose und 75,000 g Fructose sowie folgende Elektrolyte

| NaCl | 2,338 g |
|---|---|
| Na-Acetat · 3 $H_2O$ | 5,443 g |
| $CaCl_2$ · 2 $H_2O$ | 0,588 g |
| $MgCl_2$ · 6 $H_2O$ | 1,084 g |
| $ZnCl_2$ | 0,009 g |
| KCl | 0,720 g |

zugesetzt. Nachdem der pH-Wert der Lösung mit L-Äpfelsäure auf 4,5—5,0 eingestellt war, wurde mit aqua ad infundi auf ein Volumen von 1 l aufgefüllt. Die Lösungen wurden bei einer Temperatur von 80° C sterilisiert.

Diese Lösung kann auch als Lösung mit 4% Aminosäuregehalt formuliert werden, indem 40% aller Bestandteile in Wasser gelöst und auf 1 l aufgefüllt werden.

# 0 046 167

## Beispiel 2

Wie in Beispiel 1 beschrieben wird eine Nährlösung folgender Zusammensetzung bereitet, die ebenfalls bei 80°C sterilisiert wird. Das Tyrosin wird als Acetyl-L-tyrosin eingesetzt.

| | |
|---|---|
| L-Isoleucin | 3,500 g |
| L-Leucin | 6,000 g |
| L-Lysin | 6,500 g |
| L-Methionin | 1,900 g |
| L-Phenylalanin | 5,000 g |
| L-Threonin | 4,200 g |
| L-Tryptophan | 1,500 g |
| L-Valin | 6,000 g |
| L-Arginin | 8,000 g |
| L-Histidin | 6,000 g |
| L-Alanin | 16,000 g |
| L-Asparaginsäure | 2,100 g |
| L-Cystein | 0,390 g |
| L-Glutaminsäure | 9,000 g |
| Glycin | 6,000 g |
| L-Ornithin | 2,500 g |
| L-Prolin | 9,000 g |
| L-Serin | 4,500 g |
| L-Tyrosin | 1,500 g |
| L-Carnitin | 4,000 g |
| Fructose | 150,000 g |
| NaCl | 2,165 g |
| Na-Acetat · s $H_2O$ | 5,850 g |
| $CaCl_2$ · 2 $H_2O$ | 0,506 g |
| $MgCl_2$ · 6 $H_2O$ | 0,932 g |
| $ZnCl_2$ | 0,008 g |
| KCl | 0,770 g |

Äpfelsäure zur Einstellung des pH-Wertes aqua ad infundi auf 1000 ml.

## Beispiel 3

Analog Beispiel 1 wird eine Lösung mit 4% Aminosäuregehalt hergestellt, die bei 120°C sterilisiert wird und folgende Zusammensetzung hat:

| | |
|---|---|
| L-Isoleucin | 1,400 g |
| L-Leucin | 2,400 g |
| L-Lysin | 2,600 g |
| L-Methionin | 0,760 g |
| L-Phenylalanin | 2,000 g |
| L-Threonin | 1,680 g |
| L-Tryptophan | 0,600 g |
| L-Valin | 2,400 g |
| L-Arginin | 3,200 g |
| L-Histidin | 2,400 g |
| L-Alanin | 6,400 g |
| L-Asparaginsäure | 0,840 g |
| L-Cystein | 0,160 g |
| L-Glutaminsäure | 3,600 g |
| Glycin | 2,400 g |
| L-Ornithin | 1,000 g |
| L-Prolin | 3,600 g |
| L-Serin | 1,800 g |
| L-Tyrosin | 0,600 g |
| L-Carnitin | 1,600 g |
| Sorbit | 60,000 g |
| NaCl | 2,766 g |
| Na-Acetat · 3 $H_2O$ | 4,680 g |
| $CaCl_2$ · 2 $H_2O$ | 0,353 g |
| $MgCl_2$ · 6 $H_2O$ | 0,651 g |

5

| | |
|---|---|
| ZnCl$_2$ | 0,006 g |
| KCl | 0,537 g |

L-Äpfelsäure zur pH-Einstellung aqua ad infundi ad 1000 ml.

## Patentansprüche für die Vertragsstaaten: BE, CH, DE, FR, GB, IT, LI, LU, NL, SE

1. Nährlösung für die vollständige parenterale Ernährung und für gesteigerte Energieproduktion, insbesondere zur ausreichenden Aminosäureversorgung in der Postaggressionsphase mit einem Gehalt an Zucker, Aminosäuren und L-Carnitin, dadurch gekennzeichnet, daß sie in wäßriger Lösung auf 25 Gewichtsteile eines Aminosäuregemisches, bestehend aus

| | |
|---|---|
| 0,8—1,05 Gewichtsteilen | L-Isoleucin |
| 1,4—1,5 Gewichtsteilen | L-Leucin |
| 1,4—1,65 Gewichtsteilen | L-Lysin |
| 0,5—1,2 Gewichtsteilen | L-Methionin |
| 1,25—1,4 Gewichtsteilen | L-Phenylalanin |
| 0,7—1,05 Gewichtsteilen | L-Threonin |
| 0,35—0,375 Gewichtsteilen | L-Tryptophan |
| 1,0—1,5 Gewichtsteilen | L-Valin |
| 2,0—2,8 Gewichtsteilen | L-Arginin |
| 1,0—1,5 Gewichtsteilen | L-Histidin |
| 3,5—4,0 Gewichtsteilen | L-Alanin |
| 0,35—0,55 Gewichtsteilen | L-Asparaginsäure |
| 0,1 Gewichtsteil | L-Cystein |
| 2,0—2,25 Gewichtsteilen | L-Glutaminsäure |
| 1,5—2,3 Gewichtsteilen | Glycin |
| 0—0,625 Gewichtsteilen | L-Ornithin |
| 2,2—2,8 Gewichtsteilen | L-Prolin |
| 1,1—1,4 Gewichtsteilen | L-Serin |
| 0—1,5 Gewichtsteilen | L-Tyrosin, |

wobei die Aminosäuren L-Tyrosin und L-Cystein in der Lösung in Form von Acylderivaten vorliegen, maximal 50 Gewichtsteile Glucose oder Glucoseersatzstoffe, wie Fructose und Zuckeralkohole oder eines Gemisches von Glucose und Glucoseersatzstoffen und 0,8—1,2 Gewichtsteile L-Carnitin in Form des inneren Salzes oder eines pharmazeutisch verträglichen Säureadditionssalzes enthält.

2. Nährlösung nach Anspruch 1, dadurch gekennzeichnet, daß sie außerdem Elektrolyte enthält.

3. Nährlösung nach Anspruch 2, dadurch gekennzeichnet, daß die Elektrolyte Na$^+$, Ca$^{++}$, Mg$^{++}$, Zn$^{++}$, K$^+$, Chlorion und Acetation sind.

4. Nährlösung nach den Ansprüchen 2 und 3, dadurch gekennzeichnet, daß die Elektrolytmenge rund 2,5 Gewichtsteile pro 25 Gewichtsteile an Aminosäuren beträgt.

5. Verfahren zur Herstellung von Nährlösungen gemäß den Ansprüchen 1 bis 4, dadurch gekennzeichnet, daß die Aminosäuren bzw. Acylderivate dieser Aminosäuren sowie das L-Carnitin-Salz gemeinsam oder aufeinanderfolgend in warmem Wasser gelöst werden, wobei die Menge an L-Carnitin-Salz so bemessen ist, daß pro 25 Gewichtsteilen des Aminosäurengemisches 0,8—1,2 Gewichtsteile L-Carnitin zugegen sind, worauf der so entstandenen Lösung pro 25 Gewichtsteile Aminosäuregemisch maximal 50 Gewichtsteile Glucose oder Glucoseersatzstoffe, wie Fructose und Zuckeralkohole oder Mischungen von Glucose und Glucoseersatzstoffen, sowie gegebenenfalls Elektrolyte einverleibt werden und die Lösung nach gegebenenfalls erforderlicher Einstellung eines pH-Wertes innerhalb des physiologischen Bereiches mit Wasser auf das gewünschte Volumen aufgefüllt wird.

## Patentansprüche für den Vertragsstaat: AT

1. Verfahren zur Herstellung einer Nährlösung für die vollständige parenterale Ernährung und für gesteigerte Energieproduktion, insbesondere zur ausreichenden Aminosäureversorgung in der Postaggressionsphase mit einem Gehalt an Zucker, Aminosäuren und L-Carnitin, dadurch gekennzeichnet, daß ein Gemisch von Aminosäuren, das sich pro 25 Gew.-Teilen aus

| | |
|---|---|
| 0,8—1,05 Gewichtsteilen | L-Isoleucin |
| 1,4—1,5 Gewichtsteilen | L-Leucin |
| 1,4—1,65 Gewichtsteilen | L-Lysin |
| 0,5—1,2 Gewichtsteilen | L-Methionin |
| 1,25—1,4 Gewichtsteilen | L-Phenylalanin |
| 0,7—1,05 Gewichtsteilen | L-Threonin |

0 046 167

| | |
|---|---|
| 0,35—0,375 Gewichtsteilen | L-Tryptophan |
| 1,0—1,5 Gewichtsteilen | L-Valin |
| 2,0—2,8 Gewichtsteilen | L-Arginin |
| 1,0—1,5 Gewichtsteilen | L-Histidin |
| 3,5—4,0 Gewichtsteilen | L-Alanin |
| 0,35—0,55 Gewichtsteilen | L-Asparaginsäure |
| 0,1 Gewichtsteil | L-Cystein |
| 2,0—2,25 Gewichtsteilen | L-Glutaminsäure |
| 1,5—2,3 Gewichtsteilen | Glycin |
| 0—0,625 Gewichtsteilen | L-Ornithin |
| 2,2—2,8 Gewichtsteilen | L-Prolin |
| 1,1—1,4 Gewichtsteilen | L-Serin |
| 0—1,5 Gewichtsteilen | L-Tyrosin, |

zusammensetzt, wobei die Aminosäuren L-Tyrosin und L-Cystein in Form von Acylderivaten vorliegen, sowie L-Carnitin in Form seines inneren Salzes oder eines pharmazeutisch verträglichen Säureadditionssalzes in einer Menge von 0,8—1,2 Gewichtsteilen L-Carnitin auf 25 Gew.-Teile des Aminosäuregemisches gemeinsam oder aufeinanderfolgend in warmem Wasser gelöst werden, worauf der so entstandenen Lösung pro 25 Gewichtsteile Aminosäuregemisch maximal 50 Gewichtsteile Glucose oder Glucoseersatzstoffe wie Fructose und Zuckeralkohole oder Mischungen von Glucose mit den Glucoseersatzstoffen einverleibt werden und die Lösung nach gegebenenfalls erforderlicher Einstellung eines pH-Wertes innerhalb des physiologischen Bereiches mit Wasser auf das gewünschte Volumen aufgefüllt wird.

2. Verfahren nach Anspruch 1, dadurch gekennzeichnet, daß außerdem Elektrolyte zugesetzt werden.

3. Verfahren nach Anspruch 2, dadurch gekennzeichnet, daß als Elektrolyte $Na^+$, $Ca^{++}$, $Mg^{++}$, $Zn^{++}$, $K^+$, Chlorion und Acetation eingesetzt werden.

4. Verfahren nach den Ansprüchen 2 und 3, dadurch gekennzeichnet, daß die Elektrolytmenge rund 2,5 Gewichtsteile pro 25 Gewichtsteile an Aminosäuren beträgt.


**Claims for the contracting States: BE, CH, DE, FR, GB, IT, LI, LU, NL, SE**

1. Nutrient solution for complete parenteral nutrition and for increased energy production, in particular for adequate supply of amino acids in the post-insult phase, containing sugars, amino acids and L-carnitine, which is characterised in that it contains, in aqueous solution, a maximum of 50 parts by weight of glucose or glucose substitutes, such as fructose and sugar alcohols, or a mixture of glucose and glucose substitutes, and 0.8—1.2 parts by weight of L-carnitine, in the form of the inner salt or of a pharmaceutically tolerated acid addition salt, per 25 parts by weight of a mixture of amino acids consisting of

| | |
|---|---|
| 0.8—1,05 parts by weight of L-isoleucine | |
| 1.4—1.5 parts by weight of L-leucine | |
| 1.4—1.65 parts by weight of L-lysine | |
| 0.5—1.2 parts by weight of L-methionine | |
| 1.25—1.4 parts by weight of L-phenylalanine | |
| 0.7—1.05 parts by weight of L-threonine | |
| 0.35—0.375 parts by weight of L-tryptophan | |
| 1.0—1.5 parts by weight of L-valine | |
| 2.0—2.8 parts by weight of L-arginine | |
| 1.0—1.5 parts by weight of L-histidine | |
| 3.5—4.0 parts by weight of L-alanine | |
| 0.35—0.55 parts by weight of L-aspartic acid | |
| 0.1 part by weight of L-cysteine | |
| 2.0—2.25 parts by weight of L-glutamic acid | |
| 1.5—2.3 parts by weight of glycine | |
| 0—0.625 parts by weight of L-ornithine | |
| 2.2—2.8 parts by weight of L-proline | |
| 1.1—1.4 parts by weight of L-serine | |
| 0—1.5 parts by weight of L-tyrosine, | |

where the amino acids L-tyrosine and L-cystein are present in the solution in the form of acyl derivatives.

2. Nutrient solution according to Claim 1, characterised in that it also contains electrolytes.

3. Nutrient solution according to Claim 2, characterised in that the electrolytes are $Na^+$, $Ca^{++}$,

7

Mg++, Zn++, K+, chloride ions and acetate ions.

4. Nutrient solution according to Claims 2 and 3, characterised in that the amount of electrolytes is about 2.5 parts by weight per 25 parts by weight of amino acids.

5. Process for the preparation of nutrient solutions according to Claims 1—4, characterised in that the amino acids or acyl derivatives of these amino acids and the L-carnitine salt are dissolved, together or consecutively, in warm water, the amount of L-carnitine salt being such that 0.8—1.2 parts by weight of L-carnitine are present per 25 parts by weight of the amino acid mixture, and then a maximum of 50 parts by weight of glucose or glucose substitutes, such as fructose and sugar alcohols, or mixtures of glucose and glucose substitutes, per 25 parts by weight of amino acid mixture, and where appropriate, electrolytes are incorporated into the solution thus produced, and the solution is, after adjustment if necessary to a pH within the physiological range, made up to the desired volume with water.

## Claims for the contracting State AT

1. Process for the preparation of a nutrient solution for complete parenteral nutrition and for increased energy production, in particular for an adequate supply of amino acids in the post-insult phase, containing sugars, amino acids and L-carnitine, characterised in that a mixture of amino acids, which is composed of

0.8—1,05 parts by weight of L-isoleucine
1.4—1.5 parts by weight of L-leucine
1.4—1.65 parts by weight of L-lysine
0.5—1.2 parts by weight of L-methionine
1.25—1.4 parts by weight of L-phenylalanine
0.7—1.05 parts by weight of L-threonine
0.35—0.375 parts by weight of L-tryptophan
1.0—1.5 parts by weight of L-valine
2.0—2.8 parts by weight of L-arginine
1.0—1.5 parts by weight of L-histadine
3.5—4.0 parts by weight of L-alanine
0.35—0.55 parts by weight of L-aspartic acid
0.1 part by weight of L-cysteine
2.0—2.25 parts by weight of L-glutamic acid
1.5—2.3 parts by weight of glycine
0—0.625 parts by weight of L-ornithine
2.2—2.8 parts by weight of L-proline
1.1—1.4 parts by weight of L-serine
0—1.5 parts by weight of L-tyrosine,

per 25 parts by weight, the amino acids L-tyrosine and L-cysteine being present in the form of acyl derivatives, and L-carnitine, in the form of its inner salt or a pharmaceutically tolerated acid addition salt, in an amount of 0.8—1.2 parts by weight of L-carnitine to 25 parts by weight of the amino acid mixture, are dissolved, together or consecutively, in warm water, and then a maximum of 50 parts by weight of glucose or glucose substitutes, such as fructose and sugar alcohols, or mixtures of glucose with the glucose substitutes, per 25 parts by weight of amino acid mixture are incorporated into the solution thus produced, and the solution is, after adjustment if necessary to a pH within the physiological range, is made up to the desired volume with water.

2. Process according to Claim 1, characterised in that electrolytes are also added.

3. Process according to Claim 2, characterised in that the electrolytes used are Na+, Ca++, Mg++, Zn++, K+, chloride ions and acetate ions.

4. Process according to Claims 2 and 3, characterised in that the amount of electrolytes is about 2.5 parts by weight per 25 parts by weight of amino acids.

## Revendications pour les Etats contractants: BE, CH, DE, FR, GB, IT, LI, LU, NL, SE

1. Solution nutritive pour la nutrition parentérale complète et pour une production d'énergie accrue, en particulier pour une alimentation suffisante en amino-acides en phase de post-aggression, contenant du sucre, des aminoacides et de la L-carnitine, caractérisée en ce qu'elle contient, en solution aqueuse, pour 25 parties en poids d'un mélange d'amino-acides constitué par:

0,8—1,5 partie en poids de L-isoleucine
1,4—1,5 partie en poids de L-leucine
1,4—1,65 partie en poids de L-lysine

0 046 167

0,5—1,2 partie en poids de L-méthionine
1,25—1,4 partie en poids de L-phénylalanine
0,7—1,05 partie en poids de L-thréonine
0,35—0,375 partie en poids de L-tryptophane
1,0—1,5 partie en poids de L-valine
2,0—2,8 parties en poids de L-arginine
1,0—1,5 partie en poids de L-histidine
3,5—4,0 parties en poids de L-alanine
0,35—0,55 partie en poids d'acide L-aspartique
0,1 partie en poids de L-cystéine
2,0—2,25 parties en poids d'acide L-glutamique
1,5—2,3 parties en poids de glycine
0—0,625 partie en poids de L-ornithine
2,2—2,8 parties en poids de L-proline
1,1—1,4 partie en poids de L-sérine
0—1,5 partie en poids de L-tyrosine,

les amino-acides L-tyrosine et L-cystéine contenus dans la solution étant présents sous forme de dérivés acylés, au maximum 50 parties en poids du glucose ou de succédanés du glucose, comme le fructose et les alcools du sucre, ou d'un mélange de glucose ou de succédanés du glucose, et de 0,8 à 1,2 partie en poids de L-carnitine sous forme du sel interne ou d'un sel d'addition avec un acide pharmaceutiquement acceptable.

2. Solution nutritive suivant la revendication 1, caractérisée en ce qu'elle renferme en outre des électrolytes.

3. Solution nutritive suivant la revendication 2, caractérisée en ce que les électrolytes sont Na+, Ca++, Mg++, Zn++, K+, l'ion chlore et l'ion acétate.

4. Solution nutritive suivant les revendications 2 et 3, caractérisée en ce que la quantité d'électrolytes représente environ 2,5 parties en poids pour 25 parties en poids d'amino-acides.

5. Procédé pour la préparation de solutions nutritives suivant les revendications 1 à 4, caractérisé en ce qu'on fait dissoudre les amino-acides ou les dérivés acylés de ces amino-acides, ainsi que le sel de L-carnitine, de façon simultanée ou successive, dans l'eau chaude, la quantité en sel de L-carnitine étant mesurée de telle sorte que, pour 25 parties en poids du mélange d'amino-acides, 0,8 à 1,2 partie en poids de L-carnitine soit présente, après quoi on incorpore à la solution ainsi produite, pour 25 parties en poids de mélange d'amino-acides, au maximum 50 parties en poids de glucose ou de succédanés du glucose, comme le fructose et les alcools du sucre, ou de mélanges de glucose et de succédanés du glucose, ainsi que le cas échéant des électrolytes, et on amène la solution, après réglage éventuellement nécessaire d'un pH à l'intérieur de la gamme physiologique au volume désiré avec de l'eau.

## Revendications pour l'Etat contractant: AT

1. Procédé pour la préparation d'une solution nutritive pour la nutrition parentérale complète et pour une production d'énergie accrue, en particulier pour une alimentation suffisante en amino-acides en phase de post-aggression, contenant du sucre, des amino-acides et de la L-carnitine, caractérisé en ce qu'on fait dissoudre un mélange d'amino-acides contenant, pour 25 parties en poids:

0,8—1,5 partie en poids de L-isoleucine
1,4—1,5 partie en poids de L-leucine
1,4—1,65 partie en poids de L-lysine
0,5—1,2 partie en poids de L-méthionine
1,25—1,4 partie en poids de L-phénylalanine
0,7—1,05 partie en poids de L-thréonine
0,35—0,375 partie en poids de L-tryptophane
1,0—1,5 partie en poids de L-valine
2,0—2,8 parties en poids de L-arginine
1,0—1,5 partie en poids de L-histidine
3,5—4,0 parties en poids de L-alanine
0,35—0,55 partie en poids d'acide L-aspartique
0,1 partie en poids de L-cystéine
2,0—2,25 parties en poids d'acide L-glutamique
1,5—2,3 parties en poids de glycine
0—0,625 partie en poids de L-ornithine

9

2,2—2,8 parties en poids de L-proline
1,1—1,4 partie en poids de L-sérine
0—1,5 partie en poids de L-tyrosine,

les amino-acides L-tyrosine et L-cystéine étant présents sous forme de leurs dérivés acylés, ainsi que de la L-carnitine sous forme de son sel interne ou d'un sel d'addition avec un acide pharmaceutiquement acceptable, selon une quantité de 0,8 à 1,2 partie en poids de L-carnitine pour 25 parties en poids de mélange d'amino-acides, simultanément ou successivement, dans l'eau chaude, après quoi on incorpore à la solution ainsi préparée, pour 25 parties en poids du mélange d'amino-acides, au maximum 50 parties en poids de glucose ou de succédanés du glucose comme le fructose et les alcools du sucre, ou de mélanges de glucose avec des succédanés du glucose, et on complète la solution, éventuellement après réglage nécessaire d'un pH à l'intérieur de la gamme physiologique, jusqu'au volume désiré avec de l'eau.

2. Procédé suivant la revendication 1, caractérisée en ce qu'on ajoute en outre des électrolytes.

3. Procédé suivant la revendication 2, caractérisée en ce qu'on utilise comme électrolytes Na+, Ca++, Mg++, Zn++, K+, l'ion chlore et l'ion acétate.

4. Procédé suivant les revendications 2 et 3, caractérisée en ce que la quantité d'électrolytes représente environ 2,5 parties en poids pour 25 parties en poids d'amino-acides.